# FASCICULE DE BREVET EUROPEEN

(11) **EP 2 807 985 B1**
(45) Date de publication et mention de la délivrance du brevet: **02.12.2015**
(21) Numéro de dépôt: 14169218.6
(22) Date de dépôt: 21.05.2014
(51) Int. Cl.: A61B 17/80

(54) **Plaque humérale**
Humerusplatte
Humeral plate

(30) Priorité: 29.05.2013 FR 1354859
(43) Date de publication de la demande: 03.12.2014
(73) Titulaire: FX Solutions, 01000 Bourg en Bresse (FR)
(72) Inventeur: Angeloni, Renzo, 50124 Firenze (IT); Jean-Jacques, Martin, 01000 Bourg en Bresse (FR)
(74) Mandataire: Jeannet, Olivier

(56) Documents cités:
- WO-A1-2012/003884
- US-A1- 2005 240 187
- US-A1- 2007 265 629
- US-A1- 2008 300 637
- US-A1- 2010 125 300

## Description

La présente invention concerne une plaque humérale, destinée à être placée sur le côté externe d'un humérus fracturé et à être fixée à cet humérus pour permettre la consolidation de la fracture.

Il est bien connu de placer une plaque à os sur le côté externe d'un humérus fracturé afin de permettre la consolidation de la fracture. Une telle plaque comprend une partie diaphysaire allongée, sensiblement rectiligne et de largeur sensiblement constante, et une partie métaphysaire élargie par rapport à la partie diaphysaire, notamment en forme de palette, apte à recevoir des vis destinées à être insérées dans la tête humérale.

Les plaques existantes ont pour inconvénient de ne pas être très bien adaptées à la réalisation d'un repositionnement parfaitement anatomique des parties d'os fracturées. Un repositionnement non parfaitement anatomique de ces parties d'os peut avoir des conséquences notables sur la reconstruction de l'articulation.

La publication de demande de brevet N° US 2008/300637 décrit précisément une plaque tibiale mais qui peut également être une plaque humérale. Cette plaque est vrillée et a une partie métaphysaire élargie et en creux, faisant saillie à angle droit par rapport à la partie diaphysaire.

Le publication de demande de brevet N° WO 2012/003884 décrit une plaque à humérus, vrillée et à partie métaphysaire courbe, simplement élargie par rapport à la partie diaphysaire.

Les publications de demandes de brevet N° US 2010/125300, N° US 2007/265629, N° US 2008/195240 et N° WO 2004/024009, décrivent des plaques autres qu'humérales, de formes sans rapport avec celle objet de la présente demande de brevet.

La présente invention a pour but de remédier à l'inconvénient essentiel précité des plaques existantes.

La plaque humérale concernée comprend, de manière connue en soi par le premier document antérieur précité, une partie diaphysaire allongée et une partie métaphysaire élargie par rapport à la partie diaphysaire, cette plaque étant vrillée selon son axe longitudinal, de telle sorte qu'un plan médian de la partie métaphysaire et un plan médian de la partie diaphysaire forment entre eux un angle de vrillage, ce vrillage étant tel que, pour une plaque destinée à un humérus droit, la partie diaphysaire est, lorsque la plaque est vue en bout, côté proximal, décalée dans le sens horaire par rapport à la partie métaphysaire, et que, pour une plaque destinée à un humérus gauche, la partie diaphysaire est, lorsque la plaque est vue en bout, côté proximal, décalée dans le sens antihoraire par rapport à la partie métaphysaire.

Selon l'invention,
- ledit angle de vrillage compris entre 5 et 15 degrés, et
- la partie métaphysaire se projette latéralement par rapport à la partie diaphysaire, c'est-à-dire présente un axe médian longitudinal non parallèle à l'axe médian longitudinal de la partie diaphysaire, mais formant, du côté de cette partie diaphysaire, un angle d'inclinaison de 120 à 150 degrés avec l'axe médian longitudinal de la partie diaphysaire.

Il sera compris que par "plan médian", il est fait référence à un plan passant par la mi-épaisseur de l'enveloppe de la partie diaphysaire et de la partie métaphysaire, respectivement.

Ainsi, la plaque selon l'invention présente une partie métaphysaire élargie qui est vrillée longitudinalement par rapport à la partie diaphysaire. La plaque conformée de cette manière s'avère très bien adaptée à la réalisation d'un repositionnement parfaitement anatomique des parties d'os fracturées, la partie métaphysaire venant parfaitement plaquer contre la zone métaphysaire correspondante d'un humérus, pendant que la partie diaphysaire elle-même plaque également parfaitement contre la zone diaphysaire correspondante de l'humérus.

En outre, et en combinaison avec les autres caractéristiques mentionnées, la partie métaphysaire se projette latéralement par rapport à la partie diaphysaire en formant, du côté de cette partie diaphysaire, un angle d'inclinaison de 120 à 150 degrés avec l'axe médian longitudinal de la partie diaphysaire, ce qui permet d'obtenir parfaitement le résultat recherché.

De préférence, la partie métaphysaire présente une forme concave sur sa face destinée à venir en contact de l'os.

Ainsi, schématiquement, la plaque selon l'invention pourrait être définie comme "une sorte de cuillère peu profonde" dont la partie élargie est "vrillée longitudinalement par rapport au manche de la cuillère" et "tordue latéralement". La combinaison de cette forme concave de la partie métaphysaire et des autres caractéristiques de la plaque mentionnées précédemment renforce l'adaptation de la plaque à la réalisation d'un repositionnement parfaitement anatomique des parties d'os fracturées

De préférence, ladite forme concave est sensiblement en portion de sphère creuse, d'un rayon de l'ordre de 30 à 40 mm.

De préférence, ledit angle de vrillage est de l'ordre de 10 degrés.

De préférence, la plaque comprend une partie intermédiaire entre la partie métaphysaire et la partie diaphysaire, qui est vrillée selon ledit angle de vrillage.

Ce vrillage est ainsi présent au niveau de cette partie intermédiaire et non au niveau de la partie métaphysaire et de la partie diaphysaire elles-mêmes.

De préférence, cette partie intermédiaire est inclinée par rapport à la partie diaphysaire, en direction du côté de la plaque opposé à celui destiné à venir en contact avec un humérus, un axe longitudinal de cette partie intermédiaire formant un angle de l'ordre de 10 à 15° avec l'axe longitudinal de la partie diaphysaire.

De préférence, ledit angle d'inclinaison est de l'ordre de 135 degrés.

De préférence, la plaque comprend, au niveau de la partie métaphysaire, et/ou au niveau de la partie intermédiaire lorsque cette partie intermédiaire est présente, au moins un trou destiné à recevoir un ou plusieurs fils de suture, et présente, au niveau de sa face destinée à venir en contact avec un humérus, un évidement aménagé dans l'épaisseur de la partie métaphysaire et/ou de la partie intermédiaire, mettant en communication ce trou et le bord de tranche de la plaque.

Cet évidement permet le passage du ou des fils de suture entre la plaque et la paroi de l'humérus et empêche ainsi qu'un écrasement de ce ou ces fils ne se produise lorsque la plaque est appliquée contre l'os. Ces fils permettent, le cas échéant, de réinsérer des fragments d'os, en particulier les tubérosités, qui sont fréquemment fracturées et séparées du reste de l'os.

De préférence, la plaque comprend des trous à vis taraudés, de forme cylindrique, qui présentent des fentes radiales sur leur circonférence.

Ces trous sont destinés à recevoir des vis à têtes filetées de forme conique, et sont conformes à la demande de brevet N° FR 11 54791, au nom de la demanderesse.

La plaque peut également comprendre au moins un trou lisse de réception ajustée d'une broche de positionnement provisoire de la plaque contre l'os, ce trou étant notamment situé au niveau de la base de la partie métaphysaire.

L'invention sera bien comprise, et d'autres caractéristiques et avantages de celle-ci apparaîtront, en référence au dessin schématique annexé, représentant, à titre d'exemple non limitatif, une forme de réalisation préférée de la plaque humérale concernée.
La figure 1 en est une vue en perspective, par son côté destiné à venir au contact de la paroi d'un humérus ;
la figure 2 en est une vue de face ;
la figure 3 en est une vue de côté ;
la figure 4 en est une vue en bout, par son extrémité proximale ;
les figures 5A, 5B et 5C sont des vues schématiques de la plaque, expliquant le vrillage que présente cette plaque entre sa partie diaphysaire et sa partie métaphysaire ;
la figure 6 est une vue de la plaque de côté, après sa mise en place sur un humérus fracturé ;
la figure 7 en est une vue de face, également après sa mise en place sur un humérus fracturé ; et
la figure 8 représente deux plaques homologues, dont les parties diaphysaires n'ont pas la même longueur ni le même nombre de trous que la plaque montrée sur les figures 1 à 7.

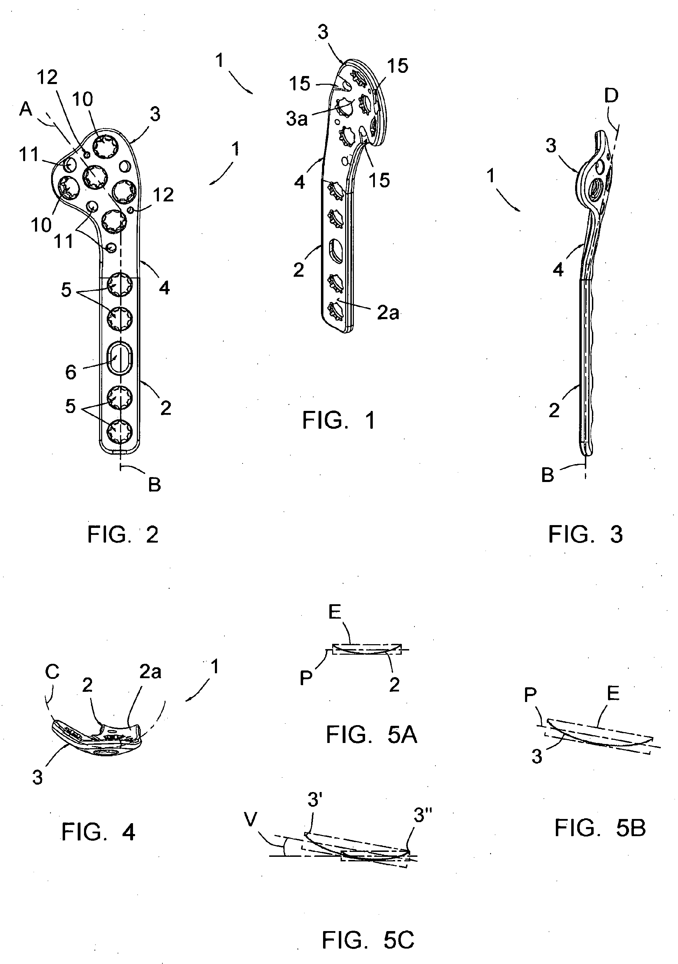

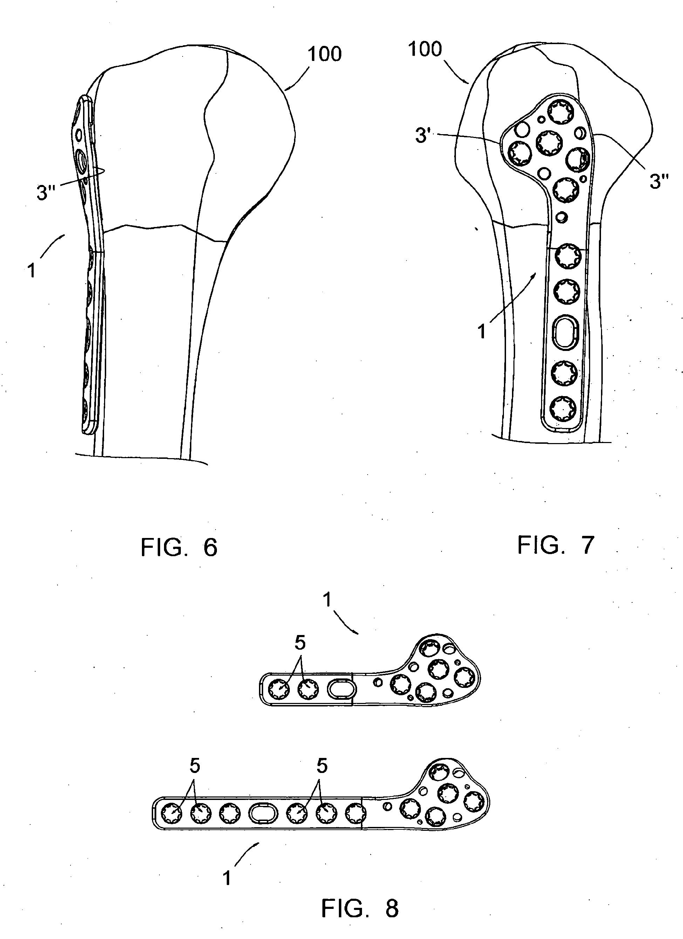

Les figures 1 à 4 représentent une plaque humérale 1, destinée, ainsi que le montrent les figures 6 et 7, à être placée sur le côté externe d'un humérus fracturé 100 et à être fixée à cet humérus par des vis (non représentées) pour permettre la consolidation de la fracture.

La plaque 1 comprend une partie diaphysaire 2 allongée, une partie métaphysaire 3 et une partie 4 intermédiaire entre ces parties 2 et 3.

La partie diaphysaire 2 est allongée et est sensiblement rectiligne et de largeur constante. Comme visible sur la figure 4, elle présente une forme arquée en section transversale, conférant à sa face 2a destinée à venir au contact de la paroi de l'humérus 100 une forme concave, adaptée à la forme arrondie de cette paroi.

La partie diaphysaire 2 comprend quatre trous à vis taraudés 5, de forme cylindrique, présentant des fentes radiales sur leur circonférence. Ces trous 5 sont destinés à recevoir des vis à têtes filetées de forme conique, et sont conformes à la demande de brevet N° FR 11 54791, au nom de la demanderesse. Il sera possible de se référer à cette demande de brevet pour de plus amples détails concernant ces trous 5.

La partie diaphysaire 2 comprend en outre un trou oblong 6 de réception d'une vis supplémentaire. Cette forme oblongue permet un réglage de la position longitudinale et/ou de l'inclinaison de cette vis supplémentaire.

La partie métaphysaire 3 est élargie par rapport à la partie diaphysaire 2 et présente une forme vaguement triangulaire, à extrémités arrondies. Comme le montre la figure 1, elle se projette latéralement par rapport à la partie diaphysaire 2, de telle sorte que son axe médian longitudinal A forme, du côté de cette partie diaphysaire 2, un angle d'inclinaison de 135 degrés avec l'axe médian longitudinal B de la partie diaphysaire 2.

Il apparaît sur les figures 1, 3 et 4 que la partie métaphysaire 3 présente une forme concave sur sa face 3a destinée à venir en contact de l'humérus 100, et que cette forme concave est sensiblement en portion de sphère creuse C. Le rayon de cette sphère creuse C est de l'ordre de 30 à 40 mm.

La partie métaphysaire 3 présente des trous à vis 10 identiques aux trous 5, trois trous 11 destinés à recevoir un ou plusieurs fils de suture, et deux trous lisses 12, de réception ajustée de broches (non représentées), de positionnement provisoire de la plaque 1 contre l'os 100.

Comme le montre particulièrement la figure 1, la partie métaphysaire 3 présente, au niveau de sa face 3a, des évidements 15 aménagés dans l'épaisseur de la partie métaphysaire 3, mettant en communication chaque trou 11 et le bord de tranche de la plaque 1. Ces évidements 15 permettent le passage du ou des fils de suture entre la plaque 1 et la paroi de l'humérus 100 et empêchent ainsi qu'un écrasement de ce ou ces fils ne se produise lorsque la plaque 1 est appliquée contre l'os. Ces fils permettent, le cas échéant, de réinsérer des fragments d'os, en particulier les tubérosités, qui sont fréquemment fracturées et séparées du reste de l'os.

La partie intermédiaire 4 est vrillée selon l'axe longitudinal de la plaque 1, d'un angle de vrillage de l'ordre de 10° entre la partie diaphysaire 2 et la partie métaphysaire 3. Précisément, comme visible sur les figures 5A à 5C, un plan médian P de la partie métaphysaire 3 (figurée par une simple courbe) et un plan médian P de la partie diaphysaire 2 (id.) forment entre eux ledit angle de vrillage V, et le bord latéral 3' de la partie métaphysaire 3 destiné à se trouver sur le côté postérieur d'un humérus 100 est plus en saillie par rapport audit plan médian P de la partie diaphysaire 2 que le bord latéral 3" de la partie métaphysaire destiné à se trouver sur le côté antérieur d'un humérus (voire également figures 6 et 7).

Il sera compris que "plan médian" se réfère à un plan passant par la mi-épaisseur de l'enveloppe E de la partie diaphysaire 2 et de la partie métaphysaire 3, respectivement.

La partie intermédiaire 4 est en outre, comme visible sur la figure 3, inclinée par rapport à la partie diaphysaire 2, en direction du côté de la plaque 1 opposé à celui destiné à venir en contact avec un humérus 100, un axe longitudinal D de cette partie intermédiaire 4 formant un angle de l'ordre de 10 à 15° avec l'axe longitudinal B de la partie diaphysaire 2.

Comme cela apparaît de ce qui précède, la plaque 1 peut être schématiquement définie comme "une sorte de cuillère peu profonde", dont la partie élargie est "tordue latéralement par rapport au manche de la cuillère " et est "vrillée longitudinalement par rapport à ce manche". La plaque 1 ainsi conformée s'avère très bien adaptée à la réalisation d'un repositionnement parfaitement anatomique des parties d'os fracturées, la partie métaphysaire 3 venant, par la combinaison de sa forme concave et de son vrillage par rapport à la partie diaphysaire 2, parfaitement plaquer contre la zone métaphysaire correspondante d'un humérus 100, pendant que la partie diaphysaire 2 elle-même plaque également parfaitement contre la zone diaphysaire correspondante de cet humérus 100.

Les deux plaques 1 montrées sur la figure 8 diffèrent de la plaque 1 montrée sur les figures 1 à 7 par la longueur de leurs parties diaphysaires 2 et par le nombre de trous 5 que comprennent ces parties diaphysaires. Elles forment, avec la plaque 1 montrée sur les figures 1 à 7, un ensemble de trois plaques pouvant être utilisées au choix selon les caractéristiques de l'os à traiter.

L'invention fournit ainsi une plaque humérale présentant des avantages déterminants par rapport aux plaques homologues selon la technique antérieure.

L'invention a été décrite ci-dessus en référence à une forme de réalisation fournie à titre d'exemple. Il va de soi qu'elle n'est pas limitée à cette forme de réalisation mais qu'elle s'étend à toutes les autres formes de réalisations couvertes par les revendications annexées.

## Revendications

1. Plaque humérale (1), destinée à être placée sur le côté externe d'un humérus (100) fracturé et à être fixée à cet humérus pour permettre la consolidation de la fracture, comprenant une partie diaphysaire (2) allongée et une partie métaphysaire (3) élargie par rapport à la partie diaphysaire, cette plaque étant vrillée selon son axe longitudinal, de telle sorte qu'un plan médian (P) de la partie métaphysaire (3) et un plan médian (P) de la partie diaphysaire (2) forment entre eux un angle de vrillage (V), ce vrillage étant tel que, pour une plaque (1) destinée à un humérus droit, la partie diaphysaire (2) est, lorsque la plaque est vue en bout, côté proximal, décalée dans le sens horaire par rapport à la partie métaphysaire (3), et que, pour une plaque (1) destinée à un humérus gauche, la partie diaphysaire (2) est, lorsque la plaque est vue en bout, côté proximal, décalée dans le sens antihoraire par rapport à la partie métaphysaire (3) ;
**caractérisée en ce que** :
- ledit angle de vrillage (V) compris entre 5 et 15 degrés, et
- la partie métaphysaire (3) se projette latéralement par rapport à la partie diaphysaire (2), c'est-à-dire présente un axe médian longitudinal (A) non parallèle à l'axe médian longitudinal (B) de la partie diaphysaire (2), mais formant, du côté de cette partie diaphysaire (2), un angle d'inclinaison de 120 à 150 degrés avec l'axe médian longitudinal (B) de la partie diaphysaire (2).

2. Plaque humérale (1) selon la revendication 1, **caractérisée en ce que** la partie métaphysaire (3) présente une forme concave sur sa face (3a) destinée à venir en contact de l'os.

3. Plaque humérale (1) selon la revendication 2, **caractérisée en ce que** ladite forme concave est sensiblement en portion de sphère creuse (C), d'un rayon de l'ordre de 30 à 40 mm.

4. Plaque humérale (1) selon l'une des revendications 1 à 3, **caractérisée en ce que** ledit angle de vrillage (V) est de l'ordre de 10 degrés.

5. Plaque humérale (1) selon l'une des revendications 1 à 4, **caractérisée en ce qu'**elle comprend une partie (4) intermédiaire entre la partie métaphysaire (3) et la partie diaphysaire (2), qui est vrillée selon ledit angle de vrillage (V).

6. Plaque humérale (1) selon la revendication 5, **caractérisée en ce que** ladite partie intermédiaire (4) est inclinée par rapport à la partie diaphysaire (2), en direction du côté de la plaque (1) opposé à celui destiné à venir en contact avec un humérus (100), un axe longitudinal (D) de cette partie intermédiaire (4) formant un angle de l'ordre de 10 à 15° avec l'axe longitudinal (B) de la partie diaphysaire (2).

7. Plaque humérale (1) selon l'une des revendications 1 à 6, **caractérisée en ce que** ledit angle d'inclinaison est de l'ordre de 135 degrés.

8. Plaque humérale (1) selon l'une des revendications 1 à 7, **caractérisée en ce qu'**elle comprend, au niveau de la partie métaphysaire (3), et/ou au niveau de la partie intermédiaire (4) lorsque cette partie intermédiaire est présente, au moins un trou (11) destiné à recevoir un ou plusieurs fils de suture, et **en ce qu'**elle présente, au niveau de sa face destinée à venir en contact avec un humérus (100), un évidement (15) aménagé dans l'épaisseur de la partie métaphysaire (3) et/ou de la partie intermédiaire (4), mettant en communication ce trou (11) et le bord de tranche de la plaque (1).

9. Plaque humérale (1) selon l'une des revendications 1 à 8, **caractérisée en ce qu'**elle comprend des trous à vis (5, 10) taraudés, de forme cylindrique, présentant des fentes radiales sur leur circonférence.

## Patentansprüche

1. Humerusplatte (1), die dazu vorgesehen ist, auf der Außenseite eines gebrochenen Humerus (100) platziert zu werden und an diesem Humerus befestigt zu werden, um die Festigung der Fraktur zu erlauben, umfassend einen verlängerten diaphysären Teil (2) und einen metaphysären Teil (3), der im Verhältnis zu dem diaphysären Teil verbreitert ist, wobei diese Platte gemäß ihrer Längsachse dergestalt verdreht ist, dass eine Mittelebene (P) des metaphysären Teils (3) und eine Mittelebene (P) des diaphysären Teils (2) untereinander einen Winkel der Verdrehung (V) bilden, wobei diese Verdrehung sich dergestalt darstellt, dass bei einer Platte (1) die für einen rechten Humerus vorgesehen ist, der diaphysäre Teil (2) auf der proximalen Seite, vom Ende der Platte aus gesehen, im Uhrzeigersinn im Verhältnis zu dem metaphysären Teil (3) versetzt ist, und bei einer Platte (1), die für einen linken Humerus vorgesehen ist, der diaphysäre Teil (2) auf der proximalen Seite, vom Ende der Platte aus gesehen, entgegen dem Uhrzeigersinn im Verhältnis zu dem metaphysären Teil (3) versetzt ist;
**dadurch gekennzeichnet, dass**:
- der Winkel der Verdrehung (V) im Bereich zwischen 5 und 15 Grad liegt, und
- der metaphysäre Teil (3) im Verhältnis zu dem diaphysären Teil (2) seitlich hervorspringt, das bedeutet eine Mittellängsachse (A) aufweist, die im Verhältnis zu der Mittellängsachse (B) des diaphysären Teils (2) nicht-parallel ist, aber auf der Seite dieses diaphysären Teils (2) einen Neigungswinkel von 120 bis 150° mit der Mittellängsachse (B) des diaphysären Teils (2) bildet.

2. Humerusplatte (1) nach Anspruch 1, **dadurch gekennzeichnet, dass** der metaphysäre Teil (3) auf seiner Seite (3a) eine konkave Form aufweist, die dazu vorgesehen ist, mit dem Knochen in Kontakt zu gelangen.

3. Humerusplatte (1) nach Anspruch 2, **dadurch gekennzeichnet, dass** die konkave Form im Wesentlichen abschnittsweise eine Hohlkugel (C) mit einem Radius in der Größenordnung von 30 bis 40 mm aufweist.

4. Humerusplatte (1) nach einem der Ansprüche 1 bis 3, **dadurch gekennzeichnet, dass** der Winkel der Verdrehung (V) in der Größenordnung von 10 Grad ist.

5. Humerusplatte (1) nach einem der Ansprüche 1 bis 4, **dadurch gekennzeichnet, dass** sie einen Zwischenteil (4) zwischen dem metaphysären Teil (3) und dem diaphysären Teil (2) umfasst, der gemäß dem Winkel der Verdrehung (V) verdreht ist.

6. Humerusplatte (1) nach Anspruch 5, **dadurch gekennzeichnet, dass** der Zwischenteil (4) im Verhältnis zu dem diaphysären Teil (2) in Richtung der Seite der Platte (1) geneigt ist, die derjenigen gegenüberliegt, die mit einem Humerus (100) in Kontakt gelangt, wobei eine Längsachse (D) dieses Zwischenteils (4) einen Winkel in der Größenordnung von 10 bis 15° mit der Längsachse (B) des diaphysären Teils (2) ausbildet.

7. Humerusplatte (1) nach einem der Ansprüche 1 bis 6, **dadurch gekennzeichnet, dass** der Neigungswinkel in der Größenordnung von 135 Grad ist.

8. Humerusplatte (1) nach einem der Ansprüche 1 bis 7, **dadurch gekennzeichnet, dass** sie in Höhe des metaphysären Teils (3) und/oder in Höhe des Zwischenteils (4), wenn dieser Zwischenteil vorhanden ist, mindestens ein Loch (11) aufweist, das dazu vorgesehen ist, einen oder mehrere Nahtfäden aufzunehmen, und **dadurch gekennzeichnet, dass** sie in Höhe ihrer Seite, die dazu vorgesehen ist, mit einem Humerus (100) in Kontakt zu gelangen, eine Aussparung (15) aufweist, die in der Dicke des metaphysären Teils (3) und/oder des Zwischenteils (4) ausgestaltet ist, wodurch dieses Loch (11) und der Rand des Abschnitts der Platte (1) miteinander in Verbindung gebracht werden.

9. Humerusplatte (1) nach einem der Ansprüche 1 bis 8, **dadurch gekennzeichnet, dass** sie gebohrte Schraubenlöcher (5, 10) zylindrischer Form umfasst, die an ihrem Umfang radiale Schlitze aufweisen.

## Claims

1. Humeral plate (1), intended to be placed on the outer side of a fractured humerus (100) and to be attached to this humerus so as to allow the healing of the fracture, comprising an elongate diaphyseal portion (2) and a metaphyseal portion (3) widened relative to the diaphyseal portion, said plate being twisted along its longitudinal axis, such that a median plane (P) of the metaphyseal portion (3) and a median plane (P) of the diaphyseal portion (2) form between them a twist angle (V), this twisting being such that, for a plate (1) for a right humerus, the diaphyseal portion (2) is, when the plate is viewed by an end, on the proximal side, shifted clockwise relative to the metaphyseal portion (3), and that, for a plate (1) for a left humerus, the diaphyseal portion (2) is, when the plate is viewed by an end, on the proximal side, shifted counterclockwise relative to the metaphyseal portion (3);
**characterized in that**:
- said twist angle (V) ranges between 5 and 15 degrees, and
- the metaphyseal portion (3) projects laterally with respect to the diaphyseal portion (2), that is to say has a longitudinal central axis (A) non-parallel to the longitudinal median axis (B) of the diaphyseal portion (2), but forming, on the side of this diaphyseal portion (2), an inclination angle of 120 to 150 degree with the longitudinal central axis (B) of the diaphyseal portion (2).

2. Humeral plate (1) according to claim 1, **characterized in that** the metaphyseal portion (3) has a concave shape on its face (3a) intended to come into contact with the bone.

3. Humeral plate (1) according to claim 2, **characterized in that** said concave shape is substantially a portion of a hollow sphere (C), which radius is ranges between about 30 to 40 mm.

4. Humeral plate (1) according to one of claims 1 to 3, **characterized in that** said twist angle (V) is about 10 degrees.

5. Humeral plate (1) according to one of claims 1 to 4, **characterized in that** it comprises a portion (4) intermediate between the metaphyseal portion (3) and the diaphyseal portion (2), which is twisted along said twist angle (V).

6. Humeral plate (1) according to claim 5, **characterized in that** said intermediate portion (4) is inclined with respect to the diaphyseal portion (2) toward the side of the plate (1) opposite that intended to come contacted with a humerus (100), a longitudinal axis (D) of this intermediate part (4) forming an angle of about 10 to 15 ° with the longitudinal axis (B) of the diaphyseal portion (2).

7. Humeral plate (1) according to one of claims 1 to 6, **characterized in that** said inclination angle is of about 135 degrees.

8. Humeral plate (1) according to one of claims 1 to 7, **characterized in that** it comprises, at the metaphyseal portion (3) and / or at the intermediate portion (4) when that part intermediate is present, at least one hole (11) for receiving one or more suture threads, and **in that** it has, at its face intended to come into contact with a humerus (100), a recess (15) formed in the thickness of the metaphyseal portion (3) and / or the intermediate part (4), by which said hole (11) communicates with the side edge of the plate (1).

9. Humeral plate (1) according to one of claims 1 to 8, **characterized in that** it comprises tapped screw holes (5, 10), cylindrical in shape, having radial slits on their circumference.
